# EUROPEAN PATENT APPLICATION

(11) **EP 3 278 875 A1**
(43) Date of publication of application: **07.02.2018**
(21) Application number: 16772919.3
(22) Date of filing: 30.03.2016
(51) Int. Cl.: B01J 35/06, B01J 37/02, B01J 37/08, C07B 61/00, C07C 1/04, C07C 1/12, C07C 9/04

(54) **CATALYST TREATMENT DEVICE AND METHOD FOR MANUFACTURING SAME**

(30) Priority: 31.03.2015 JP 2015073119
(71) Applicant: Hitachi Zosen Corporation, Osaka-shi, Osaka 559-8559 (JP)
(72) Inventor: MORI, Takuma, Osaka-shi Osaka 559-8559 (JP); SHONO, Emi, Osaka-shi Osaka 559-8559 (JP); HIKAZUDANI, Susumu, Osaka-shi Osaka 559-8559 (JP)
(74) Representative: Grünecker Patent- und Rechtsanwälte PartG mbB
(86) International application number: PCT/JP2016/060295
(87) International publication number: WO 2016/159038

(57) **Abstract**

Provided are a catalyst treatment device and a method of manufacturing the catalyst treatment device. In the catalyst treatment device, the catalyst component can be used in a smaller amount and at a lower cost without need of equipment such as casing, and can suppress excessive pressure loss with adequate voids occurring when the supported catalyst is loaded for use. The catalyst treatment device of the present invention includes a supported catalyst having a corrugated and fragmentary form, wherein the supported catalyst includes a glass paper having a corrugated and fragmentary form, a catalyst activity component supported on the glass paper and having catalytic action, and an inorganic binder necessary to cause the catalyst activity component to be supported on the glass paper and make the glass paper into a corrugated form.

## Description

### Technical Field

The present invention relates to a device used for catalyst treatment, for example, methane production by reaction of hydrogen and a mixture gas composed mainly of carbon monoxide or carbon dioxide, hydrogen production by steam reforming or ammonia decomposition, or purification of an exhaust gas, and a method of manufacturing the same.

### Background Art

To detoxify exhaust gas or other environmentally and biologically harmful gases or to convert carbon monoxide or carbon dioxide into usable materials such as hydrogen or hydrocarbon, catalyst reaction using various catalysts according to the applications is commonly used, and various forms of catalysts are commonly used for such reaction.

For example, Patent Literature 1 discloses a catalyst used for methanation reaction of carbon oxides.

When the treatment is performed on a mixture gas of carbon monoxide and carbon dioxide, the catalyst produced by the method disclosed in Patent Literature 1 can be used to construct an ideal mechanism. That is, hydrogen provided at the same time can be used to first convert all of carbon monoxide having toxicity into methane and then covert carbon dioxide into methane with the remaining hydrogen.

Patent Literature 1 discloses powdery or granular catalysts and the method of producing the same. It also discloses a method of producing the catalysts having alumina or silica as nuclei of catalyst particles. However, in the method of Patent Literature 1, the catalyst components are basically included in the inner portion of the formed material (powder or particles), and therefore, much catalyst components are needed, resulting in high costs for the catalyst production. In addition, since the methanation reaction is not slow and occurs at the surface of the catalyst, the catalyst component in the inner portion does not largely contribute to the reaction. Accordingly, in the catalyst disclosed in Patent Literature 1, not the whole catalyst component in the catalyst is effectively used.

Such a problem occurring for powdery catalysts can be overcome by forming the catalyst into a catalyst-supporting honeycomb structure. In the catalyst-supporting honeycomb structure, catalyst activity component can be supported only on the support surface having honeycomb structure, and therefore, the amount of used catalyst component per catalyst surface area (contact area or reaction area) can be reduced.

However, for conventionally known catalysts having catalyst-supporting honeycomb structure, a supporting honeycomb structure is formed integrally and then the catalyst activity component is supported thereon. Therefore, a large facility is necessary to cause the catalyst activity component to be supported uniformly. In addition, such a catalyst-supporting honeycomb structure is firmly integrated and cannot be divided, and therefore, when clogging occurs in the honeycomb structure with use, the operation for recovery has to be performed for each whole honeycomb structure and is difficult.

The Inventors of the present application have presented a supported catalyst that can overcome the problem of the conventional catalyst-supporting honeycomb structure. In this supported catalyst, corrugated glass paper supports and flat glass paper supports are stacked alternately so as not to be adhered to each other. With such a form, the operation to cause the catalyst component to be supported can be performed for each of corrugated glass papers and the flat glass papers, and thus a large facility is unnecessary. When clogging occurs in the inner portion with use, the corrugated glass papers and the flat glass papers can be separately recovered. Therefore, the problem of the conventional catalyst-supporting honeycomb structure can be overcome.

However, the supported catalyst disclosed in Patent Literature 2 is loaded into a casing so as to form and retain a honeycomb structure, and therefore, the casing is necessary for the use of the supported catalyst.

Supported catalysts having a form other than powder, granule, or honeycomb structure mentioned above are also known, and examples thereof include cylinder, ring, and pellet. However, supported catalysts having a form other than the honeycomb structure causes a large pressure loss because adequate voids do not occur when the supported catalyst is loaded, depending on the use condition.
Patent Literature 1: Japanese Patent No. 5353952
Patent Literature 2: Japanese Patent Application Publication No. 2014-117649

### Disclosure of Invention

### Technical Problem

Supported catalysts of various forms have conventionally been known. As described above, each of these supported catalysts has an advantage and a problem. In related arts, no supported catalysts have been found that can be used in a smaller amount and at a lower cost without need of equipment such as casing, and can suppress excessive pressure loss with adequate voids occurring when the supported catalyst is loaded for use.

The present invention addresses the above problems, and one object thereof is to provide a catalyst treatment device including a supported catalyst that overcomes all the problems described above and a method of manufacturing the catalyst treatment device.

### Solution to Problem

To overcome the above problems, the Applicant completed the following invention as a result of investigation. The present invention is a catalyst treatment device including a supported catalyst, wherein the supported catalyst has a corrugated and fragmentary form and includes a sheet of glass paper having a corrugated and fragmentary form, a catalyst activity component supported on the glass paper and having catalytic action, and an inorganic binder necessary to make the glass paper into a corrugated form.

The supported catalyst is preferably loaded into the catalyst treatment device randomly (irregularly).

The supported catalyst is preferably selected from the group consisting of a methanation reaction catalyst, a reforming reaction catalyst, an ammonia decomposition catalyst, and a catalyst for purification of an exhaust gas.

The inorganic binder is preferably at least one selected from the group consisting of a sol containing inorganic metal oxides and organic and inorganic salts of these metals.

The inorganic metal oxides are preferably selected from silica, alumina, titania, zirconia, yttria, lanthania, or ceria.

The organic and inorganic salts of the metals are preferably selected from an acetate, oxalate, ammonium carbonate, or nitrate of the metals.

The supported catalyst having the corrugated and fragmentary form preferably has a corrugated sectional shape including one or more repeated portions each having a width of 2.0 mm to 100 mm and a height of 1.0 mm to 50.0 mm.

The supported catalyst having the corrugated and fragmentary form preferably includes one or more corrugated portions defined by a width and a height and has a depth of 3.0 mm to 200 mm.

Further, the present invention is a method of manufacturing the above catalyst treatment device, the method comprising: applying a slurry onto a flat sheet of glass paper, the slurry containing an inorganic binder and a catalyst activity component; placing the flat sheet of glass paper having the slurry applied thereto on a die having a corrugated shape and preheated, pressing the flat sheet of glass paper against the die by a pressing jig having a shape corresponding to the corrugated shape of the die, and heating the die to a surface temperature of 100 to 500°C, so as to dehydrate and dry a surface of the glass paper and shape the glass paper into the corrugated shape, thereby forming a supported catalyst having the corrugated shape; removing the corrugated supported catalyst from the die; calcinating the removed supported catalyst having the corrugated shape to pyrolyze and eliminate an organic binder contained in the supported catalyst and oxidize the catalyst activity component; and cutting the corrugated supported catalyst into fragments each constituting a supported catalyst having a corrugated and fragmentary form.

### Advantageous Effects of Invention

In the supported catalyst included in the catalyst treatment device of the present invention, the catalyst activity component is supported only on the surface of the glass paper serving as a support. Therefore, the amount of the catalyst activity component used is smaller than in a powdery catalyst in which the catalyst activity component extends to the interior of the catalyst, resulting in a lower cost. The supported catalyst included in the catalyst treatment device of the present invention has a corrugated and fragmentary form and is required only to be loaded into the catalyst treatment device. Therefore, there is no need of providing equipment such as a casing. Since the individual supported catalyst has a corrugated shape, adequate voids occur when the supported catalyst is loaded, and excessive pressure loss can be suppressed.

### Brief Description of Drawings

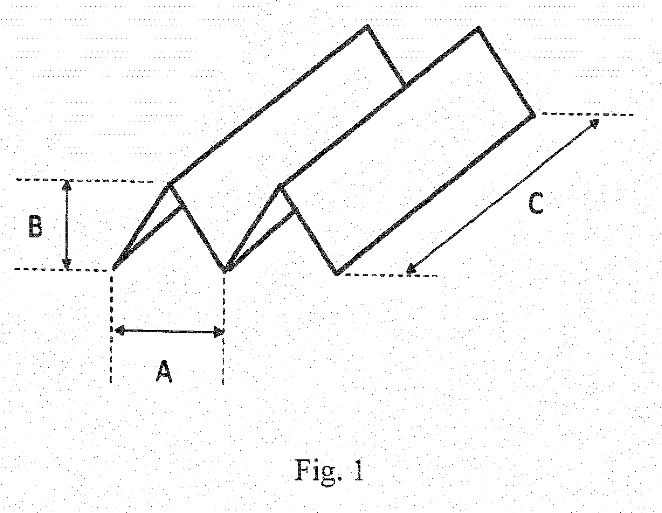

Fig. 1 is a perspective view schematically illustrating a form of a supported catalyst related to the present invention.

### Description of Embodiment

Following is description of a catalyst treatment device including a supported catalyst according to the present invention and a method of manufacturing the catalyst treatment device.

The catalyst treatment device of the present invention includes a supported catalyst, and this supported catalyst has a corrugated and fragmentary form, which is not found in conventional supported catalysts.

The form of the supported catalyst will be further described.

Fig. 1 is a perspective view schematically illustrating a form of a supported catalyst related to the present invention. As shown in Fig. 1, the supported catalyst has a corrugated shape including one or more repeated grooves. The supported catalyst has a fragmentary shape with small values for width (denoted by A) of one groove, the number of repetition of the width (n), the height (denoted by B), and the depth (denoted by C).

In such a supported catalyst, the width (A) may be not less than 2.0 mm, preferably not less than 3.0 mm, and more preferably not less than 4.0 mm. The width (A) may preferably be not greater than 100 mm, more preferably not greater than 50.0 mm, more preferably not greater than 25.0 mm and still more preferably not greater than 10.0 mm. The height (B) may be not less than 1.0 mm, preferably not less than 2.0 mm, and more preferably not less than 3.0 mm. The height (B) may preferably be not greater than 50.0 mm, more preferably not greater than 25.0 mm, and still more preferably not greater than 10.0 mm. The number of repetitions (n) in the widthwise direction may be from 1 to 100, more preferably 1 to 10, more preferably 1 to 5, and more preferably 2 to 4.

The depth (C) may be not less than 3.0 mm, preferably not less than 4.0 mm, and more preferably not less than 5.0 mm. The depth (C) may preferably be not greater than 200 mm, more preferably not greater than 100 mm, still more preferably not greater than 50.0 mm, still more preferably not greater than 20.0 mm, still more preferably not greater than 15.0 mm, and still more preferably not greater than 10.0 mm.

In the supported catalyst having the number of repetitions (n) in the widthwise direction, the width (A), the height (B), and the depth (C) may be set randomly within the above ranges.

The supported catalyst is constituted by a glass paper having a corrugated and fragmentary form. The glass paper may preferably be unwoven fabric made of glass fibers. The unwoven fabric is made of fibers not woven but entangled together into a sheet. The preferable amount of fiber may be 50 to 200 g/m².

Since glass paper is an inexpensive material, it is commonly suitable as a support of catalyst. However, it has been known that commercially available glass paper includes an organic binder which makes the commercially available glass paper difficult to work. More specifically, when a pressing force applied onto the glass paper is small, a repulsive force produced by the organic binder causes the glass paper to recover its original state. However, when the pressing force applied onto the glass paper is too large, the glass paper may be broken. Therefore, to shape the glass paper by application of the pressing force, the pressing force suited therefor is mostly within a very narrow range.

The Inventors proposed use of an inorganic binder in the glass paper having poor workability such that the glass paper becomes workable (see, e.g., Japanese Patent Application Publication No. 2014-117649).

For the supported catalyst according to the present invention, the support has to be worked into a corrugated shape in an early stage, and therefore, the glass paper containing an inorganic binder which has been proposed by the Inventors is used as a support.

The inorganic binder is applied onto the support along with the catalyst activity component for the purpose of fixing the catalyst activity component on the support. However, as described above, the inorganic binder also serves to increase the mechanical strength of the glass paper as the support such that it can be worked.

Such an inorganic binder can be selected freely as long as it can provide adequate adhesion and strength in supporting the catalyst activity component and forming the corrugated shape, and in addition, it does not inhibit the performance of the catalyst activity component used.

The increased mechanical strength provided by the inorganic binder produces the effect that the glass paper can be formed into the corrugated shape and no breakage occurs while the glass paper is cut into fragments. The corrugated and fragmentary form of the present invention is obtained by use of the glass paper containing the inorganic binder.

Also, the supported catalyst of the present invention includes the catalyst activity component having catalytic action and supported on the glass paper having the above form.

The supported catalyst of the present invention can be used for various catalytic reactions in accordance with the catalyst activity component contained therein. The supported catalyst of the present invention can be used for, e.g., methanation reaction, reforming reaction, ammonia decomposition, and purification of an exhaust gas. The catalyst activity components for achieving the various catalyst reaction may include transition metals having catalytic action (e.g., metals of Groups 4 and 8 to 10), lanthanoid and alkali metals (Group 2), alkaline earth metals (Group 3), and base metals (Groups 13 to 15) having catalytic action or catalytic promoter action, and oxides of these metals.

The catalyst activity component to be supported can be selected in accordance with the target catalytic reaction. For example, for methanation reaction and reforming reaction, one or more can be selected from nickel (Ni), cobalt (Co), platinum (Pt), etc. that are catalyst metals for these reactions; for ammonia decomposition reaction, one or more can be selected from ruthenium (Ru), cobalt (Co), nickel (Ni), etc. that are ammonia decomposition catalyst metals; and for denitration reaction, one or more can be selected from titanium (Ti), vanadium (V), and tungsten (W) that are denitration catalyst metals.

Further, the supported catalyst of the present invention contains the inorganic binder that is necessary to cause the catalyst activity component to be supported on the glass paper and is necessary to make the glass paper into a corrugated and fragmentary form.

Such an inorganic binder may be, e.g., one or more inorganic metal oxides selected from silica, alumina, titania, zirconia, yttria, lanthania, or ceria.

As described above, the supported catalyst used in the catalyst treatment device of the present invention includes commercially available glass paper as a support. The commercially available glass paper is low-cost but poor in mechanical strength. The glass paper contains the inorganic binder so as to have a high mechanical strength, and thus it can be freely shaped into a corrugated shape or cut into fragments. The supported catalyst according to the present invention has the catalyst activity component on the surface of the glass paper only. When the supported catalyst according to the present invention is loaded into the catalyst treatment device, the amount of catalyst activity component used per unit contact area may be significantly smaller than that of the conventional catalyst having an interior thereof constituted by the catalyst activity component. Further, no casing is necessary in loading the supported catalyst into the catalyst treatment device, resulting in low catalyst production cost.

The supported catalyst according to the present invention has a corrugated and fragmentary form, and therefore, when the supported catalyst is loaded randomly (irregularly) into a reactor (e.g., a reaction tank having a cylindrical shape) serving as a catalyst treatment device, adequate gaps are formed naturally, and these gaps can prevent excessive pressure loss during use of the supported catalyst.

The above description was made about the supported catalyst of the present invention as sketched in Fig. 1, which is cut into a fragment at portions corresponding to the bottoms of the grooves in the corrugated shape. The supported catalyst of the present invention essentially has a corrugated and fragmentary form, and therefore, the supported catalyst of the present invention encompasses those cut into a fragment at portions not corresponding to the bottoms of the corrugation.

Further, in the present invention, a large number of supported catalysts having a corrugated and fragmentary form may be loaded into the catalyst treatment device. The large number of supported catalyst may have either the same or different sizes.

In the supported catalyst shown in Fig. 1, the corrugated shape having the same size is continued. However, the corrugated shapes adjacent to each other may not necessarily be the same and may have different sizes.

Next, the method of manufacturing the supported catalyst according to the present invention will be hereinafter described.

The supported catalyst according to the present invention is manufactured through the following steps of:
(1) applying a slurry onto a flat sheet of glass paper, the slurry containing an inorganic binder and a catalyst activity component;
(2) placing the flat sheet of glass paper having the slurry applied thereto on a die having a corrugated shape and preheated, pressing the flat sheet of glass paper against the die by a pressing jig having a shape corresponding to the corrugated shape of the die, and heating the die so as to dehydrate and dry a surface of the glass paper having the slurry applied thereto and shape the glass paper into the corrugated shape, thereby forming a supported catalyst having the corrugated shape;
(3) removing the supported catalyst having the corrugated shape from the die;
(4) calcinating the supported catalyst having the corrugated shape to pyrolyze and eliminate an organic binder contained in the supported catalyst and oxidize the catalyst activity component; and
(5) cutting the supported catalyst having the corrugated shape into fragments each constituting a supported catalyst having a corrugated and fragmentary form.

The above steps of the method of manufacturing the supported catalyst according to the present invention may not necessarily be performed in the order presented herein but can be reordered appropriately. For example, it may also be possible that the order of the step of calcinating in (4) and the step of cutting in (5) is reversed.

### < The step of applying a slurry onto a flat sheet of glass paper, the slurry containing an inorganic binder and a catalyst activity component >

The slurry used in this step is prepared by mixing a catalyst activity component with water to prepare a solution or a suspension, adding an adequate amount of sol containing an inorganic metal oxide as an inorganic binder, and mixing it until it is uniform.

The catalyst activity component used in this step includes a precursor thereof. The precursor of the catalyst activity component may be a compound prior to obtaining catalyst activity through a heating process for the catalyst activity component to be supported on a support and an optional reduction process.

The catalyst activity component is selected in accordance with the application of the catalyst to be prepared. For example, to prepare a catalyst used for the methanation reaction, one or more selected from nickel, zirconium, and samarium is used. The catalyst activity component for preparation of the slurry has various forms as along as it can be supported on a support. For example, the catalyst activity component may have the forms of inorganic or organic salt, oxide, and complex of metals constituting activity components. Among these forms, inorganic salt may be preferable since it is soluble in water.

The inorganic binder is, more specifically, at least one selected from the group consisting of a sol containing inorganic metal oxides and organic and inorganic salts of these metals. Still more specifically, the inorganic metal oxides are selected from silica, alumina, titania, zirconia, yttria, lanthania, or ceria. The organic and inorganic salts of the metals is at least one selected from an acetate, oxalate, ammonium carbonate, or nitrate of the metals.

The concentration of the catalyst activity component in the slurry may be appropriately decided so as to achieve a desired amount of catalyst activity component per unit surface area of the supported catalyst obtained when all the steps are completed.

The concentration of the sol of the inorganic metal oxides or the salts of these metals may not be required to be strict but may be rough. When the concentration is too low and consequently the amount of the sol of the inorganic metal oxides or the salts of these metals applied onto the glass paper is too small, the catalyst activity component cannot be suitably supported on the glass paper, and the mechanical strength of the glass paper cannot be increased sufficiently. On the other hand, when the concentration of the sol of the inorganic metal oxides or the salts of these metals is too high, there may be no evident advantage in supporting of the catalyst activity component and increasing of the mechanical strength, and handling of the slurry may be difficult due to increased viscosity. In view of these points, the appropriate concentration may be, e.g., within a range of 10 to 30 wt%.

Next, the slurry thus prepared is applied uniformly onto the flat sheet of glass paper.

The slurry may be applied onto the glass paper by any method known to those skilled in the art and capable of uniform application. Examples of such a method include dipping, brushing, spraying, and dripping.

### < The step of shaping >

Next, the flat sheet of glass paper having the slurry uniformly applied thereto is placed on a preheated die. In this step, the glass paper is shaped based on the corrugated shape of the die.

The preheating is aimed at smooth heating for shaping and evaporating the moisture in the slurry, and the preheating is performed at 150 to 500°C (the same as the surface temperature of the die).

After the glass paper is placed on the die, the glass paper may be pressed against the die by a pressing jig having a shape corresponding to the corrugated shape of the die. The heating process may be performed on the glass paper thus situated. The heating process may dehydrate and dry the surface of the glass paper, and the glass paper may be kept in the corrugated shape by the action of the inorganic binder and shaped into the corrugated shape.

The die for shaping the glass paper into the corrugated shape may include a repetition of the grooves having the same shape, and it may also be possible that grooves adjacent to each other have shapes different in details.

The heating process in the step of shaping is performed at such a temperature that the surface of the glass paper can be dehydrated, for example, 100 to 500°C, but the surface temperature of the die may preferably be 150 to 500°C. When the surface temperature of the die is lower than 150°C, the dehydration may not proceed satisfactorily, resulting in defective corrugated shape. On the other hand, when the surface temperature is higher than 500°C, strain may occur in the die. Further, in view of the time efficiency during manufacturing, the more preferable temperature range may be from 200 to 500°C.

The heating process time, which may be varied in accordance with the surface temperature of the die, may be for example 5 to 600 seconds.

One example of the die used in this step may be a metal panel having grooves arranged in parallel. A flat sheet of glass paper is shaped into the corrugated shape based on the grooves arranged in parallel, and therefore, the grooves may have a width of 2.0 mm or larger and a height of 1.0 mm or larger.

The bottom portions of the grooves have a radius of curvature of 0.5 to 2 mm so as to prevent the glass paper from tearing during this step.

The pressing jig contacts directly with the glass paper on the opposite side to the die. Therefore, The contact portion of the pressing jig is water-repellent. One example of the water-repellent finishing may be Teflon™ coating.

This step produces a supported catalyst including the glass paper serving as a support and the inorganic binder and the catalyst activity component supported on the surface of the glass paper. The product of this step will be hereinafter referred to as "the corrugated supported catalyst" for simplicity.

### < The step of removing the corrugated supported catalyst from the die >

Next, the corrugated supported catalyst may be removed from the die. The corrugated supported catalyst may contain the inorganic binder and thus have an increased mechanical strength, and therefore, it can be readily removed without damage.

### < The step of calcinating the corrugated supported catalyst to pyrolyze and eliminate an organic binder contained in the supported catalyst >

In this step, the corrugated supported catalyst is calcined to pyrolyze and eliminate an organic binder contained in the supported catalyst. After the heating process in this step, the corrugated form is retained because the inorganic binder remains. The process in this step causes the catalyst activity component to enter into a desirable state. The desirable state basically refer to a transition from the form of salt to the form of oxide that is a precursor of catalyst, for example, a transition from nickel nitrate to nickel oxide.

The temperature for calcinating the corrugated supported catalyst in this step, which varies depending on the application and the components of the catalyst, may be about 200 to 550°C, and the calcinating time, which also varies as with the temperature, may be about 2 to 10 hours. The firing may be performed under the condition of air circulation. In view of the time efficiency during manufacturing, the more preferable temperature may be 200°C or higher. A temperature exceeding 550°C is undesirable since catalyst activity of the catalyst activity component may be reduced at this temperature.

### < The step of cutting the corrugated supported catalyst into fragments each constituting a supported catalyst having a corrugated and fragmentary form >

In this step, the corrugated supported catalyst is cut into fragments. This step produces the supported catalyst having a corrugated and fragmentary form.

In this step, the corrugated supported catalyst may be cut into any size, and one example of the size may be 20 by 20 mm for (the direction perpendicular to the corrugated shape) by (the depth (C)).

In this step, the corrugated supported catalyst may be cut into fragments at portions corresponding to the bottoms of the grooves in the corrugated shape, so as to obtain the supported catalyst having a corrugated and fragmentary form as shown in Fig. 1. It may also be possible that the cutting operation of this step is performed such that the cutting portions do not correspond to the bottoms of the corrugated shape.

Further, in this step, the supported catalyst may be cut into a same size such that the supported catalysts have the same size, or the supported catalyst may be cut into different sizes such that the supported catalysts have different sizes.

In the shaping step described above, the die for shaping into the corrugated shape may include grooves adjacent to each other having shapes different in details. When the corrugated supported catalyst shaped with such a die is cut, the obtained supported catalysts may include corrugations adjacent to each other having different shapes.

Thus, the supported catalyst having the corrugated and fragmentary form is obtained. Additional process may be performed on the catalyst activity component in accordance with a target catalyst reaction. For example, a reduction process may be performed. The reduction process may be performed after any of the steps (1) to (5), but it may preferably be performed after the step (5).

The reduction process will cause the catalyst activity component in the form of oxide supported on the glass paper to be reduced to the form of metal.

The supported catalyst having a corrugated and fragmentary form is obtained through these steps.

In the supported catalyst according to the present invention having a corrugated and fragmentary form, the support serving as a substrate is made of glass paper, which is an inexpensive material, and there is no need of casing, and catalyst production cost for obtaining a predetermined amount of catalyst reaction (the contact area is nearly equal to the catalyst surface area) may be low.

The obtained supported catalyst having the corrugated and fragmentary form is loaded into a catalyst space in the catalyst treatment device. The supported catalyst is loaded in the same manner as in the conventional catalyst treatment devices in which powdery or particulate catalyst is loaded. Therefore, the detailed description will be omitted.

In the catalyst treatment device according to the present invention, a plurality of supported catalysts having the corrugated and fragmentary form is loaded randomly. When the supported catalysts are loaded for use, the corrugated form produces adequate three-dimensional gaps which prevent excessive pressure loss during use of the supported catalysts.

Next, the specific surface area (the surface area per unit volume (m²/m³)) and the voidage obtained in the supported catalyst of the present invention are roughly estimated for the specific values of dimensions shown in Fig. 1.

### < Case 1 >

In Case 1, the supported catalyst may include two adjacent grooves (n=2), each having a width (A) of 10 mm and a height (B) of 10 mm, and may have a depth (C) of 10 mm (2A × B × C = 20 × 10 × 10 mm).

In this case, the specific surface area may be roughly estimated to be 440 m²/m³, and when the thickness of the supported catalyst is 1 mm, the voidage is roughly estimated to be about 78%.

These rough estimates are obtained for one supported catalyst having the above dimensions. When the supported catalysts are randomly loaded in the catalyst treatment device, the specific surface area may be slightly larger than 440m²/m³ above, and the voidage may be slightly higher than 78% above. These values may vary depending on the dimensions (A, B, C) and the number of adjacent grooves (n), and the shape and size of a loading container of the catalyst treatment device, but the values of the specific surface area 440 m²/m³ and the voidage 78% may be appropriate as reference values for later calculations.

Suppose that the supported catalyst has the above dimensions and therefore the specific surface area is 440 m²/m³ and the voidage is 78%. When 500 g/m² of catalyst component is supported per reaction area, the amount of catalyst component required to ensure the contact area of 500 m² may be 500 m² × 500 g/m² × 0.001 (kg/g) = 250 kg.

On the other hand, to ensure a contact area of 500 m² using the supported catalyst in the form of cylindrical pellet having a diameter (φ) of 3 mm and a height (H) of 3 mm, and also having a specific weight of 3400 kg/m³, the filling rate obtained when the pellet catalyst is randomly loaded into a reactor may be roughly estimated to be about 60% and the specific surface area after the loading to be 1200 m²/m³. Thus, the amount of necessary pellet catalyst may be 500 m²/1200 m²/m³ = 0 .417 m³, and the amount of necessary catalyst component may be 0.417 m³ × 3400 kg/m³ = 1417 kg.

Comparison between these catalysts shows that the amount of necessary catalyst is reduced drastically (to about 18% of that for the pellet) by use of the supported catalyst having such a form as in the present invention.

Further, the voidage of the supported catalyst having such a form as in the present invention is 78% or lower, and the voidage of the pellet catalyst is 40% (1 - (the filling factor) / 100). Therefore, comparison in the voidage after loading shows that the supported catalyst having such a form as in the present invention probably increases the voidage, and as a result, the pressure loss in the loading layer is probably reduced.

### < Case 2 >

When, in Fig. 1, the width (A), the height (B), the depth (C), the number of adjacent grooves (n), and the thickness of the catalyst (t) satisfy the relationship (A, B, C, n, t) = (5, 5, 5, 2, 1), the supported catalyst has a volume of nA × B × C = 10 × 5 × 5 mm. The specific surface area and the voidage of this supported catalyst are roughly estimated to be about 890 m²/m³ and 55%, respectively.

However, when this supported catalyst is randomly loaded into the catalyst treatment device, there may be slight variation in the specific surface area and the voidage, as in Case 1.

This supported catalyst can be compared with the cylindrical pellet catalyst (a diameter φ of 3 mm × a height H of 3 mm) in the amount of necessary catalyst activity component in the same manner as in Case 1.

The voidage of Case 2 in accordance with the present invention is 55% or lower, and the voidage of the cylindrical pellet catalyst is 40%. Therefore, comparison in the voidage after loading shows that the supported catalyst having such a form as in the present invention increases the voidage, and as a result, the pressure loss in the loading layer can be reduced.

Examples of the present invention will be hereinafter described in detail. However, the present invention is not limited to Examples described below.

### < Example 1 >

The following steps (1) to (10) were consecutively performed to manufacture a supported catalyst for methanation in accordance with the present invention.
(1) 15.0 grams of hydrosol of zirconia "Zr30AH" (from Nissan Chemical Industries, Ltd. containing 30 wt% ZrO₂ and having pH of 4.0) was mixed with 1. 969 grams of crystal of samarium nitrate hexahydrate, and the mixture was stirred until it became a homogeneous creamy sludge.
(2) 19.806 grams of nickel nitrate hexahydrate was dissolved in 20 mL of pure water to prepare an aqueous nickel nitrate solution.
(3) This aqueous solution was added into the sludge of step (1) and the mixture was stirred until it became a homogeneous solution.
(4) This homogeneous solution was mixed with 13.179 grams of silica sol "Snowtex-OS" (from Nissan Chemical Industries, Ltd. containing 20 wt% SiO₂ and having pH of 2.0 to 4.0), and the mixture was stirred again until it became a homogeneous solution, thereby producing a methanation catalyst precursor slurry.
(5) The slurry was applied to a commercially available flat glass paper (200 × 300 mm) at a rate of 6,623 g/m². The glass paper contained fibers at a rate of 100 g/m² and 10 wt% acrylic resin organic binder.
(6) A corrugation applying die (300 × 300 mm) was placed on a hot plate. The corrugation applying die was constituted by a stainless steel corrugated panel including grooves arranged in parallel. Each of the grooves had a width of 7.0 mm, a height of 7.0 mm, and a radius of curvature at the bottom of 1.6 mm. The surface of the die was heated to 300°C. The flat glass paper of step (5) having the slurry applied thereon was placed on the die, and it was pressed with a pressing jig along the grooves of the die for ten seconds for shaping. Simultaneously, the glass paper was dried by dehydrating the slurry on the surface of the glass paper. Thus, the catalyst activity component was supported on the surface of the glass paper serving as a support, and the supported catalyst having the corrugated form was obtained. Next, the corrugated supported catalyst was removed from the die. The corrugated shape of the supported catalyst was retained after removing.
(7) The removed supported catalyst having the corrugated shape was calcined under the condition of air circulation at 500°C for eight hours to eliminate the organic binder component.
(8) After calcinating, the corrugated supported catalyst was cut to the size having a width of 14.0 mm corresponding to the width of the two adj acent grooves and a depth of 5.0 mm to obtain supported catalysts for the methanation reaction having a corrugated and fragmentary form. The thickness of the obtained supported catalysts was determined to be 1.0 mm.
(9) The obtained supported catalysts having the corrugated and fragmentary form were loaded into a cylindrical reaction tube having an inner diameter of 30 mm to a loading length of 2,500 mm, and the ordinary temperature air was allowed to circulate at a space velocity of 7, 200 h⁻¹ per volume of the catalyst loading layer. The pressure difference between an inlet and an outlet of the loading layer was determined.
(10) A hydrogen gas was introduced into the reactor loaded with the supported catalyst having the corrugated and fragmentary form, and a reduction process was performed at 300°C for two hours to reduce the catalyst activity component supported on the glass paper of the supported catalysts, such that the catalyst activity component had a metallic form active for the methanation reaction.

### < Example 2 >

The supported catalysts of the present invention having the corrugated and fragmentary form were manufactured in the same manner as for Example 1 except that the surface of the die was heated to 250°C in the step (6), and the supported catalysts were loaded into a catalyst treatment device.

### < Example 3 >

The supported catalysts of the present invention having the corrugated and fragmentary form were manufactured in the same manner as for Example 1 except that the surface of the die was heated to 200°C in the step (6), and the supported catalysts were loaded into a catalyst treatment device.

### < Example 4 >

The supported catalysts of the present invention having the corrugated and fragmentary form were manufactured in the same manner as for Example 1 except that the surface of the die was heated to 150°C in the step (6), and the supported catalysts were loaded into a catalyst treatment device.

### < Example 5 >

The supported catalysts of the present invention having the corrugated and fragmentary form were manufactured in the same manner as for Example 1 except that the surface of the die was heated to 100°C in the step (6), and the supported catalysts were loaded into a catalyst treatment device.

### < Comparative Example 1 >

The supported catalysts of the present invention having the corrugated and fragmentary form were manufactured in the same manner as for Example 1 except that the surface of the die was maintained at an ordinary temperature in the step (6), and the supported catalysts were loaded into a catalyst treatment device.

Table 1 below shows the time required for removal after shaping with the die having corrugated grooves and the quality of corrugated shape for Examples 1 to 5 and Comparative Example 1.

In Table 1, the time required for removal refers to the rough time from the point when the pressing jig used for shaping by pressing was taken away to the point when the corrugated supported catalyst slipped down spontaneously by gravity from the die raised obliquely. The quality of the corrugated shape was determined based on whether or not the removed catalysts retained the shape conforming to the corrugation of the die used for shaping. In Table 1, "Good" indicates "a good quality," "Poor" indicates "a poor quality," and "Medium" indicates a quality between the "good quality" and the "poor quality."

**Table 1**

| | Surface Temperature of Die (°C) | Time Required for Removal (sec) | Quality of Corrugated Shape |
|---|---|---|---|
| Example 1 | 300 | 3 | Good |
| Example 2 | 250 | 90 | Good |
| Example 3 | 200 | 300 | Good |
| Example 4 | 150 | 600 | Good |
| Example 5 | 100 | 3000 | Medium |
| Comparative Example 1 | Ordinary Temp. | - | Poor |

As shown in Table 1, the corrugated shapes of Examples 1 to 5 were good. The time required for removal was longer as the surface temperature of the die was lower. When the surface temperature of the die was 100°C as in Example 5, the time required for removal was significantly longer, resulting in low efficiency in drying. Under the condition of the surface temperature in Example 5, the corrugated supported catalysts were manufactured with a poor corrugated shape. However, these results are based on the view of efficiency in manufacturing process and the quality of the shape of the manufactured catalysts. As for catalyst performance, Example 5 was satisfactory. In Comparative Example 1, in which the surface temperature of the die was lower than in Example 5, the supported catalysts were not removed from the die and no corrugation was formed.

Next, in Examples 6 to 11 and Comparative Examples 2 to 3 below, a die including grooves with a different size was used to manufacture supported catalysts with a different size.

### < Example 6 >

The supported catalysts of the present invention having the corrugated and fragmentary form were manufactured in the same manner as for Example 1, except that the die used in the step (6) had a width (A) of 4.0 mm and a height (B) of 3.0 mm and, in the step (8), the corrugated supported catalyst was cut to the size having a width of 8.0 mm corresponding to the width of the two adjacent grooves and a depth of 5.0 mm. The supported catalysts were loaded into a catalyst treatment device.

### < Example 7 >

The supported catalysts of the present invention having the corrugated and fragmentary form were manufactured in the same manner as for Example 1, except that the die used in the step (6) had a width (A) of 3.0 mm and a height (B) of 2.0 mm and, in the step (8), the corrugated supported catalyst was cut to the size having a width of 6.0 mm corresponding to the width of the two adjacent grooves and a depth of 5.0 mm. The supported catalysts were loaded into a catalyst treatment device.

### < Example 8 >

The supported catalysts of the present invention having the corrugated and fragmentary form were manufactured in the same manner as for Example 1, except that the die used in the step (6) had a width (A) of 15.0 mm and a height (B) of 15.0 mm and, in the step (8), the corrugated supported catalyst was cut to the size having a width of 30.0 mm corresponding to the width of the two adjacent grooves and a depth of 5.0 mm. The supported catalysts were loaded into a catalyst treatment device.

### < Example 9 >

The supported catalysts of the present invention having the corrugated and fragmentary form were manufactured in the same manner as for Example 1, except that the die used in the step (6) had a width (A) of 3.0 mm and a height (B) of 2.0 mm and, in the step (8), the corrugated supported catalyst was cut to the size having a width of 6.0 mm corresponding to the width of the two adjacent grooves and a depth of 3.0 mm. The supported catalysts were loaded into a catalyst treatment device.

### < Example 10 >

The supported catalysts of the present invention having the corrugated and fragmentary form were manufactured in the same manner as for Example 1, except that the die used in the step (6) had a width (A) of 2.0 mm and a height (B) of 2.0 mm and, in the step (8), the corrugated supported catalyst was cut to the size having a width of 4.0 mm corresponding to the width of the two adjacent grooves and a depth of 5.0 mm. The supported catalysts were loaded into a catalyst treatment device.

### < Example 11 >

The supported catalysts of the present invention having the corrugated and fragmentary form were manufactured in the same manner as for Example 1, except that the die used in the step (6) had a width (A) of 2.0 mm and a height (B) of 1.0 mm and, in the step (8), the corrugated supported catalyst was cut to the size having a width of 4.0 mm corresponding to the width of the two adjacent grooves and a depth of 5.0 mm. The supported catalysts were loaded into a catalyst treatment device.

### < Comparative Example 2 >

In place of the supported catalysts manufactured in Example 1, cylindrical pellet catalysts having a diameter of 3.0 mm and a height of 3.0 mm were loaded into a catalyst treatment device.

### < Comparative Example 3 >

Nothing was loaded into a catalyst treatment device.

Table 2 below shows the voidage and the pressure difference in the loading layer of the catalyst treatment device (nothing is loaded in Comparative Example 3) for Examples 1 and 6 to 11 and Comparative Examples 2 to 3.

The voidage was obtained by (X-Y) /X, where X is the volume of the space occupied by the catalysts in the reactor, and Y is the total volume of the fragments of the loaded supported catalysts. The pressure difference was determined between an inlet and an outlet of the loading layer, as described for Example 1.

**Table 2**

| | Shape of Catalyst | Dimensions of Catalyst | | | Voidage in Loading Layer | Pressure Difference in Loading Layer |
|---|---|---|---|---|---|---|
| | | Width/ Diameter | Height | Depth | | |
| | | (mm) | (mm) | (mm) | (%) | (kPa) |
| Example 1 | Corrugated | 14.0 | 7.0 | 5.0 | 82 | 3.2 |
| Example 6 | Corrugated | 8.0 | 3.0 | 5.0 | 52 | 10.9 |
| Example 7 | Corrugated | 6.0 | 2.0 | 5.0 | 46 | 13.3 |
| Example 8 | Corrugated | 30.0 | 15.0 | 5.0 | 94 | 1.0 |
| Example 9 | Corrugated | 6.0 | 2.0 | 3.0 | 41 | 21.8 |
| Example 10 | Corrugated | 4.0 | 2.0 | 5.0 | 39 | 26.5 |
| Example 11 | Corrugated | 4.0 | 1.0 | 5.0 | 33 | 30.6 |
| Comparative Example 2 | Cylindrical | 3.0 | 3.0 | - | 35 | 33.8 |
| Comparative Example 3 | - | - | - | - | 100 | 0.07 |

The results shown in Table 2 indicates that the supported catalysts of the present invention having the corrugated and fragmentary form can be loaded into a reactor so as to achieve a high voidage in the loading layer and a low pressure loss (pressure difference) in the loading layer.

As indicated by the results of Examples 1 and 6 to 11, the voidage is lower and the pressure loss is higher as the corrugated shape is finer. In Example 11, the voidage is lower than that of Comparative Example 3 in which the cylindrical pellet catalysts were loaded, but the pressure loss is about the same as that of Comparative Example 3, indicating the advantageous effect of the corrugated and fragmentary form.

As indicated by Example 9, when the supported catalysts having the corrugated and fragmentary form have a small depth, the pressure loss of the loading layer tends to be high.

## Claims

1. A catalyst treatment device including a supported catalyst, wherein the supported catalyst has a corrugated and fragmentary form and includes a glass paper having a corrugated and fragmentary form, a catalyst activity component supported on the glass paper and having catalytic action, and an inorganic binder necessary to make the glass paper into a corrugated form.

2. The catalyst treatment device of claim 1 wherein the supported catalyst is loaded into the catalyst treatment device randomly.

3. The catalyst treatment device of claim 1 wherein the supported catalyst is selected from the group consisting of a methanation reaction catalyst, a reforming reaction catalyst, an ammonia decomposition catalyst, and a catalyst for purification of an exhaust gas.

4. The catalyst treatment device of claim 1 wherein the inorganic binder is at least one selected from the group consisting of a sol containing inorganic metal oxides and organic and inorganic salts of these metals.

5. The catalyst treatment device of claim 4 wherein the inorganic metal oxides are selected from silica, alumina, titania, zirconia, yttria, lanthania, or ceria.

6. The catalyst treatment device of claim 4 wherein the organic and inorganic salts of the metals are selected from an acetate, oxalate, ammonium carbonate, or nitrate of the metals.

7. The catalyst treatment device of claim 1 wherein the supported catalyst having the corrugated and fragmentary form has a corrugated sectional shape including one or more repeated portions each having a width of 2.0 mm to 100 mm and a height of 1.0 mm to 50.0 mm.

8. The catalyst treatment device of claim 7 wherein the supported catalyst having the corrugated and fragmentary form includes one or more corrugated portions defined by a width and a height and has a depth of 3.0 mm to 200 mm.

9. A method of manufacturing the catalyst treatment device of any one of claims 1 to 8, the method comprising:
applying a slurry onto a flat sheet of glass paper, the slurry containing an inorganic binder and a catalyst activity component;
placing the flat sheet of glass paper having the slurry applied thereto on a die having a corrugated shape and preheated, pressing the flat sheet of glass paper against the die by a pressing jig having a shape corresponding to the corrugated shape of the die, and heating the die to a surface temperature of 100 to 500°C, so as to dehydrate and dry a surface of the glass paper and shape the glass paper into the corrugated shape, thereby forming a supported catalyst having the corrugated shape;
removing the corrugated supported catalyst from the die;
calcinating the removed supported catalyst having the corrugated shape to pyrolyze and eliminate an organic binder contained in the supported catalyst and oxidize the catalyst activity component; and
cutting the corrugated supported catalyst into fragments each constituting a supported catalyst having a corrugated and fragmentary form.
